# EUROPEAN PATENT SPECIFICATION

(11) **EP 1 677 772 B1**
(45) Date of publication and mention of the grant of the patent: **26.08.2009**
(21) Application number: 03770801.3
(22) Date of filing: 29.10.2003
(51) Int. Cl.: A61K 31/00, A61K 31/616, A61P 25/24

(54) **ORAL ANTIDEPRESSANT FORMULATION COMPRISING ACETYLSALICYLIC ACID TO ACCELERATE ONSET OF ACTION**
ORALES ANTIDEPRESSIVUM ENTHALTEND ACETYLSALICYLSÄURE ZUR BESCHLEUNIGUNG DES WIRKUNGSEINTRITTS
FORMULATION ORALE A BASE D'ANTIDEPRESSEURS COMPRENANT UN ACIDE ACETYLSALICYLIQUE POUR ACCELERER SON ACTION

(43) Date of publication of application: 12.07.2006
(73) Proprietor: Kriwin, Philippe, 1180 Bruxelles (BE); Powis de Tenbossche, Roland, 1980 Eppegem (BE); Mendlewicz, Julien, 1653 Dworp (BE)
(72) Inventor: Kriwin, Philippe, 1180 Bruxelles (BE); Powis de Tenbossche, Roland, 1980 Eppegem (BE); Mendlewicz, Julien, 1653 Dworp (BE)
(74) Representative: Overath, Philippe
(86) International application number: PCT/BE2003/000181
(87) International publication number: WO 2005/039545

(56) References cited:
- WO-A-00/28980
- WO-A-00/59489
- WO-A-03/103643
- GB-A- 2 287 404
- US-A- 4 650 789
- US-B1- 6 432 989
- AYUK J ET AL: "A blind panic" LANCET THE, LANCET LIMITED. LONDON, GB, vol. 357, no. 9264, 21 April 2001 (2001-04-21), page 1262, XP004246109 ISSN: 0140-6736
- JUAREZ-OLGUIN HUGO ET AL: "Adverse effects of imipramine are increased by interaction with ASA in depressed patients." NEUROPSYCHOPHARMACOLOGY, vol. 22, no. 1, January 2000 (2000-01), pages 100-101, XP002255347 & ISSN: 0893-133X
- DE ABAJO FRANCISCO JOSE ET AL: "Association between selective serotonin reuptake inhibitors and upper gastrointestinal bleeding: Population based case-control study." BMJ, vol. 319, no. 7217, 23 October 1999 (1999-10-23), pages 1106-1109, XP002255348 & ISSN: 0959-8138

## Description

The present invention relates to a formulation and provides a method for reducing the onset of action of pharmaceutically acceptable antidepressant active agents preferably selected of the group consisting of SSRI agents, SNRIs (serotonin noradrenaline reuptake inhibitors), CRF antagonists, NK1 antagonists, NK2 antagonists, NK3 antagonists and combinations thereof.

The major problem of human antidepressant is the onset time required for obtaining an antidepressant action, said onset being often greater than 4 weeks. This means a great risk that the patient decides him self to stop the treatment in view of the lack of action after two weeks treatment, or to take a too large dose, possibly a toxic dose, of one or more antidepressant agents.

The present invention has for object an oral antidepressant formulation with a reduced time of onset of antidepressant action. It has been discovered that by using specific compound(s), it was possible to reduce the onset of antidepressant action of antidepressant drugs, such as SSRIs, as well as pharmaceutically acceptable antidepressant active agents selected of the group consisting of SNRIs (serotonin noradrenaline reuptake inhibitors), CRF antagonists, NK1 antagonists, NK2 antagonists, NK3 antagonists and combinations thereof.

The invention relates to an oral antidepressant formulation comprising an effective antidepressant amount of one or more pharmaceutically acceptable antidepressant active agents, preferably selective serotonin reuptake inhibitors (SSRI), SNRIs (serotonin noradrenaline reuptake inhibitors), CRF antagonists, NK1 antagonists, NK2 antagonists, NK3 antagonists and combinations thereof (most preferably SNRIs (serotonin noradrenaline reuptake inhibitors), CRF antagonists, NK1 antagonists, NK2 antagonists, NK3 antagonists and combinations thereof), and an effective amount lower than 50mg of at least a compound or additive selected from the group consisting of acetylsalicylic acid (ASA), salts and esters of acetylsalicylic acid (the acid form being however preferred), diaspirin, and mixtures thereof, for reducing the time of onset of antidepressant action of the pharmaceutically acceptable antidepressant active agents preferably selective serotonin reuptake inhibitor(s), SNRIs (serotonin noradrenaline reuptake inhibitors), CRF antagonists, NK1 antagonists, NK2 antagonists, NK3 antagonists and combinations thereof) ASA and derivatives thereof (such as salts, esters thereof) and mixtures thereof are however the preferred compound(s) to be combined with the SSRI compound or pharmaceutically acceptable antidepressant active agents selected of the group consisting of SNRIs (serotonin noradrenaline reuptake inhibitors), CRF antagonists, NK1 antagonists, NK2 antagonists, NK3 antagonists and combinations thereof.
The amount of aspirin, diaspirin or salt or ester of acetylsalicylic acid, and combinations thereof is lower than 50mg, preferably lower than 40mg, such as comprised between 5 and 35mg.

While the mechanism of action is not clear, it seems that the reduced onset is due to one or more of the following reasons :
- better passage of the antidepressant active agent in the blood
- higher fluidity of the blood
- increased deposited in the brain
- activation of the brain, so that the brain has a higher receptivity for the antidepressant active agent

One or more other advantages of the formulation of the invention are:
- quicker action
- less headaches
- possibility to reduce the doses of antidepressant
- less risk factor for suicide (for example due to the immediate action of the antidepressant)
The formulation comprises advantageously an effective amount of at least a compound selected from the group consisting of acetylsalicylic acid, salts and esters of acetylsalicylic acid and mixtures thereof, for reducing at least 15%, advantageously at least 20%, preferably at least 50% the average onset of action of the pharmaceutically acceptable antidepressant active agents preferably selected from the group consisting of SSRI, SNRIs (serotonin nordraline reuptake inhibitors), CRF antagonists, NK1 antagonists, NK2 antagonists, NK3 antagonists and combinations thereof, most preferably from the group consisting of SNRIs (serotonin noradrenaline reuptake inhibitors), CRF antagonists, NK1 antagonists, NK2 antagonists, NK3 antagonists and combinations thereof.
Preferably, the effective amount of one or more compounds for reducing the time of onset of antidepressant action of the pharmaceutically acceptable antidepressant active agents selected of the group consisting of SSRI, SNRIs (serotonin noradrenaline reuptake inhibitors), CRF antagonists, NK1 antagonists, NK2 antagonists, NK3 antagonists and combinations thereof. is lower than 5 times the amount of pharmaceutically acceptable antidepressant active agents selected of the group consisting of SSRI, SNRIs (serotonin noradrenaline reuptake inhibitors), CRF antagonists, NK1 antagonists, NK2 antagonists, NK3 antagonists and combinations thereof.
Most preferably, the effective amount of one or more compounds for reducing the time of onset of antidepressant action of phrmaceutically acceptable antidepressant active agents selected of the group consisting of SSRI, SNRIs (serotonin noradrenaline reuptake inhibitors), CRF antagonists, NK1 antagonists, NK2 antagonists, NK3 antagonists and combinations thereof is lower than 2 times, advantageously lower than 1.5 times, preferably lower than 1 times the amount of pharmaceutically acceptable antidepressant active agents selected of the group consisting of SSRI, SNRIs (serotonin noradrenaline reuptake inhibitors), CRF antagonists, NK1 antagonists, NK2 antagonists, NK3 antagonists and combinations thereof.
According to a preferred embodiment, the formulation comprises a quick release effective amount of at least a compound selected from the group consisting of acetylsalicylic acid, salts and esters of acetylsalicylic acid, diaspirin, and mixtures thereof, for reducing the time of onset of antidepressant action of the selective serotonin reuptake inhibitor(s).
According to a detail of a specific embodiment, the formulation comprises particles, core, microparticles, spherical particles comprising at least one selective serotonin reuptake inhibitor or a pharmaceutically acceptable antidepressant active agents selected of the group consisting of SSRI, SNRIs (serotonin noradrenaline reuptake inhibitors), CRF antagonists, NK1 antagonists, NK2 antagonists, NK3 antagonists and combinations thereof, and possibly at least a compound selected from the group consisting of acetylsalicylic acid, salts and esters of acetylsalicylic acid, and mixtures thereof, said particles, core, microparticles, spherical particles being advantageously provided with a coating comprising at least a compound selected from the group consisting of acetylsalicylic acid, salts and esters of acetylsalicylic acid, diaspirin, and mixtures thereof, for reducing the onset of action of the selective serotonin reuptake inhibitor(s) or pharmaceutically acceptable antidepressant active ag ts selected of the group consisting of SSRI, SARIs (serotonin noradrenaline reuptake inhibitors), CRF antagonists, NK1 antagonists, NK2 antagonists, NK3 antagonists and combinations thereof, said coating being substantially free of selective serotonin reuptake inhibitor or pharmaceutically acceptable antidepressant active agents selected of the group consisting of SSRI, SNRIs (serotonin noradrenaline reuptake inhibitors), CRF antagonists, NK1 antagonists, NK2 antagonists, NK3 antagonists and combinations thereof. The coating is for example made by using a water soluble polymer. It has however to be noted that in case the SSRI and the additive selected from the group consisting of acetylsalicylic acid, salts and esters of acetylsalicylic acid, and mixtures thereof, are mixed together so as to prepare matrix comprising both compounds, the additive acts as means for ensuring a better dissolution of the pharmaceutically acceptable antidepressant active agents selected of the group consisting of SSRI, SNRIs (serotonin noradrenaline reuptake inhibitors), CRF antagonists, NK1 antagonists, NK2 antagonists, NK3 antagonists and combinations thereof, possibly mixed with SSRI and/or a better passage in the organism. It can be advantageous to provide a barrier coating between the SSRI containing core, particles, etc. and the coating containing the additive, so as to avoid any interaction between the pharmaceutically acceptable antidepressant active agents selected of the group consisting of SSRI, SNRIs (serotonin noradrenaline reuptake inhibitors), CRF antagonists, NK1 antagonists, NK2 antagonists, NK3 antagonists and combinations thereof, and the additive. Such a barrier coating is for example a water insoluble coating (polymer, etc.). This is advantageous for avoiding any stability problem.
Advantageously, the composition of the invention comprises a pharmaceutically acceptable antidepressant active agents selected of the group consisting of SSRI, SNRIs (serotonin noradrenaline reuptake inhibitors), CRF antagonists, NK1 antagonists, NK2 antagonists, NK3 antagonists and combinations thereof, the SSRI (selective serotonin reuptake inhibitor) being most preferably selected from the group consisting of citalopram, especially escitalopram or the s-isomer of citalopram, paroxetine, sertraline, fluvoxamine, fluoxetine. Fluoxetine, citalopram, escitalopram and sertraline are the most preferred SSRI.
While the formulation can be liquid, the formulation is advantageously solid. Solid formulation means a formulation which has a solid aspect, even if the pharmaceutically acceptable antidepressant active agents selected of the group consisting of SSRI, SNRIs (serotonin noradrenaline reuptake inhibitors), CRF antagonists, NK1 antagonists, NK2 antagonists, NK3 antagonists and combinations thereof is in a liquid or semi liquid form in a carrier or on a carrier. For example, the solid form comprises from 4mg up to 100mg, preferably from 4 to 20mg of pharmaceutically acceptable antidepressant active agents selected of the group consisting of SSRI, SNRIs (serotonin noradrenaline reuptake inhibitors), CRF antagonists, NK1 antagonists, NK2 antagonists, NK3 antagonists and combinations thereof.

The invention relates further to an antidepressant system comprising at least a formulation, advantageously an oral formulation, with an effective antidepressant amount of one or more selective serotonin reuptake inhibitors or a pharmaceutically acceptable antidepressant active agents selected of the group consisting of SNRIs (serotonin noradrenaline reuptake inhibitors), CRF antagonists, NK1 antagonists, NK2 antagonists, NK3 antagonists and combinations thereof, and at least a formulation, advantageously an oral formulation, with an effective amount of less than 50 mg of at least a compound selected from the group consisting of acetylsalicylic acid, salts and esters of acetylsalicylic acid, diaspirin, and mixtures thereof, for reducing the time of onset of antidepressant action of the selective serotonin reuptake inhibitor(s) or pharmaceutically acceptable antidepressant active agents selected of the group consisting of SNRIs (serotonin noradrenaline reuptake inhibitors), CRF antagonists, NK1 antagonists, NK2 antagonists, NK3 antagonists and combinations thereof. The two formulations can be administered simultaneously or successively, and/or with different administration pathways (oral, rectal, parenteral, etc.) or with the same administration pathways.
The system of the invention has advantageously one or more characteristics of the oral formulation of the invention as described here above.

The invention enables to treat a patient suffering a depression, in which the patient is administered an effective antidepressant amount of one or more pharmaceutically acceptable antidepressant active agents, preferably selected of the group consisting of SSRI, SNRIs (serotonin noradrenaline reuptake inhibitors), CRF antagonists, NK1 antagonists, NK2 antagonists, NK3 antagonists and combinations thereof, such as mixtures of one or more selective serotonin reuptake inhibitors with one or more CRF and/or NK1 and/or NK2 and/or NK3 antagonists, and an effective amount of at least an additive compound selected from the group consisting of acetylsalicylic acid, salts and esters of acetylsalicylic acid, diaspirin and mixtures thereof, for reducing the time of onset of antidepressant action of the pharmaceutically acceptable antidepressant, preferably SSRI, SNRIs (serotonin noradrenaline reuptake inhibitors), CRF antagonists, NK1 antagonists, NK2 antagonists, NK3 antagonists and combinations thereof. The amount of additive (acetylsalicylic acid, salts and esters of acetylsalicylic acid, diaspirin, and mixtures thereof) is lower than 50mg for a human with a weight of 70kg, preferably comprised between 5mg and 40mg.

The doses of pharmaceutically acceptable antidepressant active agents advantageously selected of the group consisting of SSRIs, SNRIs (serotonin noradrenaline reuptake inhibitors), CRF antagonists, NK1 antagonists, NK2 antagonists, NK3 antagonists and combinations thereof to be administered to the patient are for example the doses proposed in the commercial formulation of pharmaceutically acceptable antidepressant active agents, advantageously selected from the group consisting of SSRIs, SNRIs (serotonin noradrenaline reuptake inhibitors), CRF antagonists, NK1 antagonists, NK2 antagonists, NK3 antagonists and combinations thereof or by the doctors with such commercial formulation. However, these doses can possibly be lowered, for example by a factor 2 or 3.

As it seems also that the time of antidepressant action (or time efficiency) of the drug pharmaceutically acceptable antidepressant active agents advantageously selected from the group consisting of SSRIs, SNRIs (serotonin noradrenaline reuptake inhibitors), CRF antagonists, NK1 antagonists, NK2 antagonists, NK3 antagonists and combinations thereof is increased by the additive, less doses have to be taken with respect to the commercial formulations. For example one daily dose with the formulation of the invention, while two or more dose were required for a commercial SSRI formulation, SNRIs (serotonin noradrenaline reuptake inhibitors), CRF antagonists, NK1 antagonists, NK2 antagonists, NK3 antagonists and combinations thereof.

The pharmaceutically acceptable antidepressant active agents selected of the group consisting of SNRIs (serotonin noradrenaline reuptake inhibitors), CRF antagonists, NK1 antagonists, NK2 antagonists, NK3 antagonists and combinations thereof are advantageously compounds as disclosed in the following references :
for SNRIs (serotonin noradrenaline reuptake inhibitors), CRF antagonists : US6525067; US6432989; US 6613777; US6610678; US6589958; US6589952; US6469166, etc
for NK1 antagonists : US6635630, US6096766
for NK2 antagonists : US5889024; US6444809, US6355695, US6124279; US 6008223; US5977135; US5567700; US5521199, etc.
for NK3 antagonist: US6040316; US6602886; US6348330; US6258943, etc. for combination of NK1 antagonist with NK2 antagonist: US6177450, US6147083, etc.

Examples of SNRIs are commercial products such as Venlafaxine, duloxetine, milnacipram, reboxetine, and mixtures thereof.

### EXAMPLES

### Example 1

A PROZAC solid form containing 20mg fluoxetine was coated with a thin water insoluble barrier coating. The so coated solid form was then coated with a water soluble polymer mixed with ASA (acetyl salicylic acid). The water soluble outer coating comprised about 5mg ASA.

### Examples 2 to 5

Example 1 was repeated except that the amount of ASA in the outer coating was respectively 10mg, 20mg, 40 mg.

### Examples 6 to 10

Examples 1 to 5 were repeated but with a 10mg fluoxetine solid form.

### Example 11

A solid form containing 35 mg ASA was prepared.
The form was coated with a (water insoluble) barrier coating. The so coated form was then overcoated with a layer (made of a water soluble polymer) in which 10 mg fluoxetine is dispersed. An outer protective layer is advantageously added.

### Example 12

Example 11 is repeated except that the fluoxetine containing layer contains 20mg fluoxetine.

Examples 11 and 12 have been repeated, except that the central ASA solid form contained respectively 10mg, 20mg, 40mg ASA.

It is obvious that the above formulation can be prepared by any techniques, such as spheronisation, so as to prepare microgranules or microspheres of fluoxetine coated with ASA containing layer or microgranules or microspheres of ASA coated with fluoxetine containing layer, with or without intermediate barrier layer.

Examples 1 to 12 have been repeated, except that fluoxetine was replaced by sertraline, paroxetine, citalopram, escitalopram or the s isomer of citalopram, and fluvoxamine.

### Example 13

A citalopram solid form containing 20mg citalopram was coated with a thin water insoluble barrier coating. The so coated solid form was then coated with a water soluble polymer mixed with ASA. The water soluble outer coating comprised about 5mg ASA.

### Examples 14 to 17

Example 13 was repeated except that the amount of ASA in the outer coating was respectively 10mg, 20mg,30mg and 40mg.

### Example 18

A solid form containing 35 mg ASA was prepared.
The form was coated with a (water insoluble) barrier coating. The so coated form was then overcoated with a layer (made of a water soluble polymer) in which 20 mg citalopram is dispersed. An outer protective layer is advantageously added.

### Example 19

Example 18 was repeated except that the solid form contains 25mg ASA.

### Example 20

A solid form containing 10mg escitalopram was coated with a thin water insoluble barrier coating. The so coated solid form was then coated with a water soluble polymer mixed with ASA. The water soluble outer coating comprised about 5mg ASA.

### Examples 21 to 24

Example 20 was repeated except that the amount of ASA in the outer coating was respectively 10mg, 20mg, 35 mg and 40mg.

### Example 25

A solid form containing 35 mg ASA was prepared.
The form was coated with a (water insoluble) barrier coating. The so coated form was then overcoated with a layer (made of a water soluble polymer) in which 10 mg escitalopram is dispersed. An outer protective layer is advantageously added.

### Example 26

Example 25 was repeated except that the solid form contains 25mg ASA.

### Examples 27 to 38

Example 1 to 12 have been repeated except that fluoxetine was replaced by Venlafaxin.

### Examples 39 to 74

Examples 1 to 12 have been replaced except that fluoxetine was replaced respectively by Duloxetine, milnacipram and reboxetine.

### Tests made on rats

Four groups of rats have been stressed. One group of rats remain unstressed so as to serve as control.

After being stressed, the rats of the first group received a dose of 50mg ASA per kg. The second group of rats received no treatment. The third group of rats received 5mg fluoxetine per kg, and the fourth group of rats received simultaneously 5mg fluoxetine + 50mg ASA per kg.
The treatment was continued during 3 weeks.

The control group of rats had an activity level of 30, while the stressed rats had an activity of 2.

The result of this animal test is :
- after 1 week of treatment with the combination fluoxetine+ASA, the fourth group of rats had an activity of 30, i.e. corresponding to the activity of the unstressed rats or control rats. After 1 week of treatment with ASA or fluoxetine alone, there was no increase at all of the activity.
- after three weeks of treatment, the fourth group of rats had still an activity of 30. The third group of rats (treated with fluoxetine) had after 3 weeks an activity of about 25, while no improvement of activity was observed with ASA.

In said test, the doses of ASA and fluoxetine have been adapted for rats.

Said test shows clearly the unexpected action or synergistic activity of ASA + fluoxetine. While not being bound to any theory, it is believed that ASA promotes or activates the sites on which the fluoxetine has to bind.

Further tests have been carried out with lower doses of ASA, namely 25mg. Similar onset of antidepressant action was observed.

In view of the reducing time of onset antidepressant action obtained for the family of SSRI antidepressant, it can be concluded that ASA at dose lower than 50mg is also efficient for reducing the time of antidepressant onset action for pharmaceutically acceptable antidepressant active agents selected of the group consisting of SNRIs (serotonin noradrenaline reuptake inhibitors), CRF antagonists, NK1 antagonists, NK2 antagonists, NK3 antagonists and combinations thereof, possibly mixed with one or more SSRI.

From said tests and informal tests made on volunteers, in which after (just after, less than 5 minutes after) and prior oral administration of an antidepressant, a dose of ASA was orally administered, it appears that the oral dose of ASA had to be lowered to less than 50mg, especially between 5 and 50mg.

At such a low dose of ASA, no bleeding was observed, but a very quick onset of antidepressant action was observed. From this test, it seems that the antidepressant action could already be achieved in less than 7days, and even in less than 3 to 4 days, although when not using low dose of ASA, the antidepressant action is for example of at least 4 weeks for SSRI.

Higher dose of ASA (dose of 100mg for example) administered to human seems to have no influence or even to have a negative influence on the time of onset of antidepressant action.

## Claims

1. Antidepressant system for human comprising at least a formulation, advantageously an oral formulation, with an effective antidepressant amount of one or more pharmaceutically acceptable antidepressant active agents, said agent(s) being preferably selected of the group consisting of SSRI agents, SNRIs (serotonin noradrenaline reuptake inhibitors), CRF antagonists, NK1 antagonists, NK2 antagonists, NK3 antagonists and combinations thereof, and at least a formulation, advantageously an oral formulation, with an effective amount lower than 50mg of at least a compound selected from the group consisting of acetylsalicylic acid, salts and esters of acetylsalicylic acid, diaspirin, and mixtures thereof, for reducing the antidepressant onset of action of the pharmaceutically acceptable antidepressant active agents preferably selected from the group consisting of SSRI agents, SNRIs (serotonin noradrenaline reuptake inhibitors), CRF antagonists, NK1 antagonists, NK2 antagonists, NK3 antagonists and combinations thereof.

2. Oral antidepressant formulation for human comprising an effective antidepressant amount of one or more pharmaceutically acceptable antidepressant active agents preferably selected of the group consisting of SSRI agents, SNRIs (serotonin noradrenaline reuptake inhibitors), CRF antagonists, NK1 antagonists, NK2 antagonists, NK3 antagonists and combinations thereof, and an effective amount lower than 50mg of at least a compound selected from the group consisting of acetylsalicylic acid, salts and esters of acetylsalicylic acid, diaspirin, and mixtures thereof, for reducing the onset of action of the pharmaceutically acceptable antidepressant active agents preferably selected from the group consisting of SSRI agents, SNRIs (serotonin noradrenaline reuptake inhibitors), CRF antagonists, NK1 antagonists, NK2 antagonists, NK3 antagonists and combinations thereof.

3. The formulation or system of claim 1 or 2, which comprises an effective amount of at least a compound selected from the group consisting of acetylsalicylic acid, salts and esters of acetylsaticylic acid, diaspirin, and mixtures thereof, for reducing at least 15%, advantageously at least 20%, preferably at least 50% the average onset of action of the pharmaceutically acceptable antidepressant active agents preferably selected from the group consisting of SSRI agents, SNRIs (serotonin noradrenaline reuptake inhibitors), CRF antagonists, NK1 antagonists, NK2 antagonists, NK3 antagonists and combinations thereof.

4. The formulation or system of claim 1 or 2,in which the effective amount of one or more compounds for reducing the time of onset of antidepressant action of the pharmaceutically acceptable antidepressant active agents selected of the group consisting of SSRI agents, SNRIs (serotonin noradrenaline reuptake inhibitors), CRF antagonists, NK1 antagonists, NK2 antagonists, NK3 antagonists and combinations thereof is lower than 5 times the amount of pharmaceutically acceptable antidepressant active agents preferably selected from the group consisting of SSRI agents, SNRIs (serotonin noradrenaline reuptake inhibitors), CRF antagonists, NK1 antagonists, NK2 antagonists, NK3 antagonists and combinations thereof.

5. The formulation or system of claim 1 or 2,in which the effective amount of one or more compounds for reducing the time of onset of antidepressant action of the pharmaceutically acceptable antidepressant active agents preferably selected from the group consisting of SSRI agents, SNRIs (serotonin noradrenaline reuptake inhibitors), CRF antagonists, NK1 antagonists, NK2 antagonists, NK3 antagonists and combinations thereof is lower than 1.5 times, advantageously lower than 1 times the amount of pharmaceutically acceptable antidepressant active agents preferably selected from the group consisting of SSRI agents, SNRIs (serotonin noradrenaline reuptake inhibitors), CRF antagonists, NK1 antagonists, NK2 antagonists, NK3 antagonists and combinations thereof.

6. The formulation or system of claim 1 or 2, which comprises a quick release effective amount of at least a compound selected from the group consisting of acetylsalicylic acid, salts and esters of acetylsalicylic acid, diaspirin, and mixtures thereof, for reducing the time of onset of antidepressant action of the pharmaceutically acceptable antidepressant active agents preferably selected from the group consisting of SSRI agents, SNRIs (serotonin noradrenaline reuptake inhibitors), CRF antagonists, NK1 antagonists, NK2 antagonists, NK3 antagonists and combinations thereof.

7. The formulation or system of claim 1 or 2, which comprises particles, core, microparticles, spherical particles comprising at least one pharmaceutically acceptable antidepressant active agents preferably selected from the group consisting of SSRI agents, SNRIs (serotonin noradrenaline reuptake inhibitors), CRF antagonists, NK1 antagonists, NK2 antagonists, NK3 antagonists and combinations thereof and possibly at least a compound selected from the group consisting of acetylsalicylic acid, salts and esters of acetylsalicylic acid, diaspirin, and mixtures thereof, said particles, core, microparticles, spherical particles being advantageously provided with a coating comprising at least a compound selected from the group consisting of acetylsalicylic acid, salts and esters of acetylsalicylic acid, diaspirin and mixtures thereof, for reducing the onset of action of the pharmaceutically acceptable antidepressant active agents preferably selected from the group consisting of SSRI agents, SNRIs (serotonin noradrenaline reuptake inhibitors), CRF antagonists, NK1 antagonists, NK2 antagonists, NK3 antagonists and combinations thereof, said coating being substantially free of selective pharmaceutically acceptable antidepressant active agents preferably selected from the group consisting of SSRI agents, SNRIs (serotonin noradrenaline reuptake inhibitors), CRF antagonists, NK1 antagonists, NK2 antagonists, NK3 antagonists and combinations thereof.

8. The formulation or system of claim 1 or 2, which is a solid form comprising from 4mg up to 100mg, preferably from 4 to 20mg pharmaceutically acceptable antidepressant active agents preferably selected from the group consisting of SSRI agents, SNRIs (serotonin noradrenaline reuptake inhibitors), CRF antagonists, NK1 antagonists, NK2 antagonists, NK3 antagonists and combinations thereof.

9. The formulation or system of claim 1 or 2, which comprises from 5 to 40mg acetylsalicylic acid, salts or esters of acetylsalicylic acid, diaspirin, and mixtures thereof, preferably from 5 to 35mg.

10. The formulation or system of claim 1 or 2, which comprises as pharmaceutically acceptable antidepressant agent an effective amount of an agent selected from the group consisting of SNRIs (serotonin noradrenaline reuptake inhibitors), CRF antagonists, NK1 antagonists, NK2 antagonists, NK3 antagonists and combinations thereof.

11. The formulation or system of claim 1 or 2, which comprises as pharmaceutically acceptable antidepressant agent an effective amount of one or more SSRI agents.

12. Use of a compound selected from the group consisting of acetylsalicylic acid, salts and esters of acetylsalicylic acid; diaspirin and mixture thereof for the preparation of antidepressant composition or system with a reducted time of onset of antidepressant action of an antidepressant active agent selected from the group consisting of SSRI agents, SNRIs (serotonin noradrenaline reuptake inhibitors), CRF antagonists, NK1 antagonists, NK2 antagonists, NK3 antagonists and combinations thereof.

13. The use of claim 12 for the preparation of antidepressant composition or system with less suicide risk factor associated to an antidepressant active agent selected from the group consisting of SSRI agents, SNRIs (serotonin noradrenaline reuptake inhibitors), CRF antagonists, NK1 antagonists, NK2 antagonists, NK3 antagonists and combinations thereof.

14. The use of claim 12 for the preparation of antidepressant composition or system with an increased time of action or time efficiency of an antidepressant active agent selected from the group consisting of SSRI agents, SNRIs (serotonin noradrenaline reuptake inhibitors), CRF antagonists, NK1 antagonists, NK2 antagonists, NK3 antagonists and combinations thereof.

## Patentansprüche

1. Antidepressivumsystem für Menschen, umfassend mindestens eine Formulierung, vorteilhafterweise eine Oralformulierung, mit einer wirkungsvollen antidepressiven Menge von einem oder mehreren pharmazeutisch verträglichen Antidepressivumwirkstoffen, wobei der (die) Wirkstoff(e) vorzugsweise ausgewählt ist (sind) aus der Gruppe, bestehend aus SSRI-Mitteln, SNRIs (Serotonin, Noradrenalinwiederaufnahmeinhibitoren), CRF-Antagonisten, NK1-Antagonisten, NK2-Antagonisten, NK3-Antagonisten und Kombinationen davon und mindestens eine Formulierung, vorteilhafterweise eine Oralformulierung, mit einer wirkungsvollen Menge, die geringer ist als 50 mg, von mindestens einer Verbindung, ausgewählt aus der Gruppe, bestehend aus Acetylsalicylsäure, Salzen und Estern von Acetylsalicylsäure, Diaspirin und Gemischen davon, zum Verkürzen des Einsetzens der antidepressiven Wirkung der pharmazeutisch verträglichen Antidepressivumwirkstoffe, vorzugsweise ausgewählt aus der Gruppe, bestehend aus SSRI-Mitteln, SNRIs (Serotonin, Noradrenalinwiederaufnahmeinhibitoren), CRF-Antagonisten, NK1-Antagonisten, NK2-Antagonisten, NK3-Antagonisten und Kombinationen davon.

2. Oralantidepressivumformulierung für Menschen, umfassend eine wirkungsvolle antidepressive Menge von einem oder mehreren pharmazeutisch verträglichen Antidepressivumwirkstoffen, vorzugsweise ausgewählt aus der Gruppe, bestehend aus SSRI-Mitteln, SNRIs (Serotonin, Noradrenalinwiederaufnahmeinhibitoren), CRF-Antagonisten, NK1-Antagonisten, NK2-Antagonisten, NK3-Antagonisten und Kombinationen davon, und eine effektive Menge, die geringer ist als 50 mg von mindestens einer Verbindung, ausgewählt aus der Gruppe, bestehend aus Acetylsalicylsäure, Salzen und Estern von Acetylsalicylsäure, Diaspirin und Gemischen davon, zum Verkürzen des Einsetzens der Wirkung der pharmazeutisch verträglichen Antidepressivumwirkstoffe, vorzugsweise ausgewählt aus der Gruppe, bestehend aus SSRI-Mitteln, SNRIs (Serotonin, Noradrenalinwiederaufnahmeinhibitoren), CRF-Antagonisten, NK1-Antagonisten, NK2-Antagonisten, NK3-Antagonisten und Kombinationen davon

3. Formulierung oder System nach Anspruch 1 oder 2, umfassend eine wirkungsvolle Menge mindestens einer Verbindung, ausgewählt aus der Gruppe, bestehend aus Acetylsalicylsäure, Salzen und Estern von Acetylsalicylsäure, Diaspirin und Gemischen davon, um im Mittel das Einsetzen der Wirkung der pharmazeutisch verträglichen Antidepressivumwirkstoffe um mindestens 15 %, vorzugsweise um mindestens 20 %, vorzugsweise um mindestens 50 % zu verringern, vorzugsweise ausgewählt aus der Gruppe, bestehend aus SSRI-Mitteln, SNRIs (Serotonin, Noradrenalinwiederaufnahmeinhibitoren), CRF-Antagonisten, NK1-Antagonisten, NK2-Antagonisten, NK3-Antagonisten und Kombinationen davon.

4. Formulierung oder System nach Anspruch 1 oder 2, worin die wirkungsvolle Menge von einer oder mehreren Verbindungen zum Verkürzen der Zeit des Einsetzens der antidepressiven Wirkung der pharmazeutisch verträglichen Antidepressivumwirkstoffe, ausgewählt aus der Gruppe, bestehend aus SSRI-Mitteln, SNRIs (Serotonin, Noradrenalinwiederaufnahmeinhibitoren), CRF-Antagonisten, NK1-Antagonisten, NK2-Antagonisten, NK3-Antagonisten und Kombinationen davon, geringer ist als 5mal die Menge des pharmazeutisch verträglichen Antidepressivumwirkstoffe, vorzugsweise ausgewählt aus der Gruppe, bestehend aus SSRI-Mitteln, SNRIs (Serotonin, Noradrenalinwiederaufnahmeinhibitoren), CRF-Antagonisten, NK1-Antagonisten, NK2-Antagonisten, NK3-Antagonisten und Kombinationen davon.

5. Formulierung oder System nach Anspruch 1 oder 2, worin die wirkungsvolle Menge der ein oder mehreren Verbindungen zum Verkürzen der Zeit des Einsetzens der antidepressiven Wirkung der pharmazeutisch verträglichen Antidepressivumwirkstoffe, vorzugsweise ausgewählt aus der Gruppe, bestehend aus SSRI-Mitteln, SNRIs (Serotonin, Noradrenalinwiederaufnahmeinhibitoren), CRF-Antagonisten, NK1-Antagonisten, NK2-Antagonisten, NK3-Antagonisten und Kombinationen davon, geringer als 1,5mal, vorteilhafterweise geringer als 1 mal die Menge von pharmazeutisch verträglichen Antidepressivumwirkstoffen ist, vorzugsweise ausgewählt aus der Gruppe, bestehend aus SSRI-Mitteln, SNRIs (Serotonin, Noradrenalinwiederaufnahmeinhibitoren), CRF-Antagonisten, NK1-Antagonisten, NK2-Antagonisten, NK3-Antagonisten und Kombinationen davon.

6. Formulierung oder System nach Anspruch 1 oder 2, umfassend eine für schnelle Freisetzung wirkungsvolle Menge mindestens einer Verbindung, ausgewählt aus der Gruppe, bestehend aus Acetylsalicylsäure, Salzen und Estern von Acetylsalicylsäure, Diaspirin und Gemischen davon, zum Verkürzen der Zeit des Einsetzens der antidepressiven Wirkung der pharmazeutisch verträglichen Antidepressivumwirkstoffe, vorzugsweise ausgewählt aus der Gruppe, bestehend aus SSRI-Mitteln, SNRIs (Serotonin, Noradrenalinwiederaufnahmeinhibitoren), CRF-Antagonisten, NK1-Antagonisten, NK2-Antagonisten, NK3-Antagonisten und Kombinationen davon.

7. Formulierung oder System nach Anspruch 1 oder 2, umfassend Teilchen, Kern, Mikroteilchen, sphärische Teilchen, umfassend mindestens einen pharmazeutisch verträglichen Antidepressivumwirkstoft, vorzugsweise ausgewählt aus der Gruppe, bestehend aus SSRI-Mitteln, SNRIs (Serotonin, Noradrenalinwiederaufnahmeinhibitoren), CRF-Antagonisten, NK1-Antagonisten, NK2-Antagonisten, NK3-Antagonisten und Kombinationen davon und gegebenenfalls mindestens eine Verbindung, ausgewählt aus der Gruppe, bestehend aus Acetylsalicylsäure, Salzen und Estern von Acetylsalicylsäure, Diaspirin und Gemischen davon, wobei die Teilchen, Kern, Mikroteilchen, sphärischen Teilchen vorteilhafterweise bereitgestellt werden mit einer Beschichtung, umfassend mindestens eine Verbindung, ausgewählt aus der Gruppe, bestehend aus Acetylsalicylsäure, Salzen und Estern von Acetylsalicylsäure, Diaspirin und Gemischen davon, zum Verkürzen des Einsetzens der Wirkung der pharmazeutisch verträglichen Antidepressivumwirkstoffe vorzugsweise ausgewählt aus der Gruppe, bestehend aus SSRI-Mitteln, SNRIs (Serotonin, Noradrenalinwiederaufnahmeinhibitoren), CRF-Antagonisten, NK1-Antagonisten, NK2-Antagonisten, NK3-Antagonisten und Kombinationen davon, wobei die Beschichtung im Wesentlichen frei von selektiv pharmazeutisch verträglichen Antidepressivumwirkstoffen ist, vorzugsweise ausgewählt aus der Gruppe, bestehend aus SSRI-Mitteln, SNRIs (Serotonin, Noradrenalinwiederaufnahmeinhibitoren), CRF-Antagonisten, NK1-Antagonisten, NK2-Antagonisten, NK3-Antagonisten und Kombinationen davon.

8. Formulierung oder System nach Anspruch 1 oder 2, die eine feste Form ist, umfassend von 4 mg bis zu 100 mg, vorzugsweise von 4 bis 20 mg pharmazeutisch verträgliche Antidepressivumwirkstoffe, vorzugsweise ausgewählt aus der Gruppe, bestehend aus SSRI-Mitteln, SNRIs (Serotonin, Noradrenalinwiederaufnahmeinhibitoren), CRF-Antagonisten, NK1-Antagonisten, NK2-Antagonisten, NK3-Antagonisten und Kombinationen davon.

9. Formulierung oder System nach Anspruch 1 oder 2, umfassend von 5 bis 40 mg Acetylsalicylsäure, Salze oder Ester von Acetylsalicylsäure, Diaspirin und Gemische davon, vorzugsweise von 5 bis 35 mg.

10. Formulierung oder System nach Anspruch 1 oder 2, umfassend als pharmazeutisch verträgliches Antidepressivum eine wirkungsvolle Menge eines Mittels, ausgewählt aus der Gruppe, bestehend aus SNRIs (Serotonin, Noradrenalinwiederaufnahmeinhibitoren), CRF-Antagonisten, NK1-Antagonisten, NK2-Antagonisten, NK3-Antagonisten und Kombinationen davon.

11. Formulierung oder System nach Anspruch 1 oder 2, umfassend als pharmazeutisch verträgliches Antidepressivum eine wirkungsvolle Menge von einem oder mehreren SSRI-Mitteln.

12. Verwendung einer Verbindung, ausgewählt aus der Gruppe, bestehend aus Acetylsalicylsäure, Salzen und Estern von Acetylsalicylsäure, Diaspirin und Gemischen davon, zur Herstellung einer Antidepressivumzusammensetzung oder eines Antidepressivumsystems mit einer verkürzten Zeit des Einsetzens der antidepressiven Wirkung eines Antidepressiumwirkstoffs, ausgewählt aus der Gruppe, bestehend aus SSRI-Mitteln, SNRIs (Serotonin, Noradrenalinwiederaufnahmeinhibitoren), CRF-Antagonisten, NK1-Antagonisten, NK2-Antagonisten, NK3-Antagonisten und Kombinationen davon.

13. Verwendung nach Anspruch 12 zur Herstellung einer Antidepressivumzusammensetzung oder eines Antidepressivumsystems mit einem geringeren Suizidrisikofaktor, verbunden mit einem Antidepressivumwirkstoff, ausgewählt aus der Gruppe, bestehend aus SSRI-Mitteln, SNRIs (Serotonin, Noradrenalinwiederaufnahmeinhibitoren), CRF-Antagonisten, NK1-Antagonisten, NK2-Antagonisten, NK3-Antagonisten und Kombinationen davon.

14. Verwendung nach Anspruch 12 zur Herstellung einer Antidepressivumzusammensetzung oder eines Antidepressivumsystems mit einer erhöhten Wirkzeit oder Zeiteffizienz des Antidepressivumwirkstoffs, ausgewählt aus der Gruppe, bestehend aus SSRI-Mitteln, SNRIs (Serotonin, Noradrenalinwiederaufnahmeinhibitoren), CRF-Antagonisten, NK1-Antagonisten, NK2-Antagonisten, NK3-Antagonisten und Kombinationen davon.

## Revendications

1. Système à base d'antidépresseurs pour l'homme comprenant au moins une formulation, avantageusement une formulation orale, avec une quantité antidépressive efficace d'un ou de plusieurs agents antidépresseurs actifs pharmaceutiquement acceptables, le ou lesdits agents étant de préférence choisis parmi le groupe constitué des agents ISRS, des IRSN (inhibiteurs de la recapture de la sérotonine et de la noradrénaline), des antagonistes du CRF, des antagonistes de NK1, des antagonistes de NK2, des antagonistes de NK3 et des combinaisons de ceux-ci, et au moins une formulation, avantageusement une formulation orale, avec une quantité efficace inférieure à 50 mg d'au moins un composé choisi parmi le groupe constitué de l'acide acétylsalicylique, des sels et des esters de l'acide acétylsalicylique, de la diaspirine, et des mélanges de ceux-ci, pour réduire le délai d'action antidépressive des agents antidépresseurs actifs pharmaceutiquement acceptables de préférence choisis parmi le groupe constitué des agents ISRS, des IRSN (inhibiteurs de la recapture de la sérotonine et de la noradrénaline), des antagonistes du CRF, des antagonistes de NK1, des antagonistes de NK2, des antagonistes de NK3 et des combinaisons de ceux-ci.

2. Formulation orale à base d'antidépresseurs pour l'homme comprenant une quantité antidépressive efficace d'un ou de plusieurs agents antidépresseurs actifs pharmaceutiquement acceptables, de préférence choisis parmi le groupe constitué des agents ISRS, des IRSN (inhibiteurs de la recapture de la sérotonine et de la noradrénaline), des antagonistes du CRF, des antagonistes de NK1, des antagonistes de NK2, des antagonistes de NK3 et des combinaisons de ceux-ci, et une quantité efficace inférieure à 50 mg d'au moins un composé choisi parmi le groupe constitué de l'acide acétylsalicylique, des sels et des esters de l'acide acétylsalicylique, de la diaspirine, et des mélanges de ceux-ci, pour réduire le délai d'action antidépressive des agents antidépresseurs actifs pharmaceutiquement acceptables de préférence choisis parmi le groupe constitué des agents ISRS, des IRSN (inhibiteurs de la recapture de la sérotonine et de la noradrénaline), des antagonistes du CRF, des antagonistes de NK1, des antagonistes de NK2, des antagonistes de NK3 et des combinaisons de ceux-ci.

3. Formulation ou système selon les revendications 1 ou 2, qui comprend une quantité efficace d'au moins un composé choisi parmi le groupe constitué de l'acide acétylsalicylique, des sels et des esters de l'acide acétylsalicylique, de la diaspirine, et des mélanges de ceux-ci, pour réduire d'au moins 15%, avantageusement d'au moins 20%, de préférence d'au moins 50% le délai moyen d'action des agents antidépresseurs actifs pharmaceutiquement acceptables de préférence choisis parmi le groupe constitué des agents ISRS, des IRSN (inhibiteurs de la recapture de la sérotonine et de la noradrénaline), des antagonistes du CRF, des antagonistes de NK1, des antagonistes de NK2, des antagonistes de NK3 et des combinaisons de ceux-ci.

4. Formulation ou système selon les revendications 1 ou 2, où la quantité efficace d'un ou plusieurs des composés pour réduire le temps de début de l'action antidépressive des agents antidépresseurs actifs pharmaceutiquement acceptables de préférence choisis parmi le groupe constitué des agents ISRS, des IRSN (inhibiteurs de la recapture de la sérotonine et de la noradrénaline), des antagonistes du CRF, des antagonistes de NK1, des antagonistes de NK2, des antagonistes de NK3 et des combinaisons de ceux-ci est inférieure à 5 fois la quantité des agents antidépresseurs actifs pharmaceutiquement acceptables de préférence choisis parmi le groupe constitué des agents ISRS, des IRSN (inhibiteurs de la recapture de la sérotonine et de la noradrénaline), des antagonistes du CRF, des antagonistes de NK1, des antagonistes de NK2, des antagonistes de NK3 et des combinaisons de ceux-ci.

5. Formulation ou système selon les revendications 1 ou 2, où la quantité efficace d'un ou plusieurs des composés pour réduire le temps de délai de l'action antidépressive des agents antidépresseurs actifs pharmaceutiquement acceptables de préférence choisis parmi le groupe constitué des agents ISRS, des IRSN (inhibiteurs de la recapture de la sérotonine et de la noradrénaline), des antagonistes du CRF, des antagonistes de NK1, des antagonistes de NK2, des antagonistes de NK3 et des combinaisons de ceux-ci est inférieure à 1,5 fois, avantageusement inférieure à 1 fois la quantité des agents antidépresseurs actifs pharmaceutiquement acceptables de préférence choisis parmi le groupe constitué des agents ISRS, des IRSN (inhibiteurs de la recapture de la sérotonine et de la noradrénaline), des antagonistes du CRF, des antagonistes de NK1, des antagonistes de NK2, des antagonistes de NK3 et des combinaisons de ceux-ci.

6. Formulation ou système selon les revendications 1 ou 2, qui comprend une quantité efficace à libération rapide d'au moins un composé choisi parmi le groupe constitué de l'acide acétylsalicylique, des sels et des esters de l'acide acétylsalicylique, de la diaspirine, et des mélanges de ceux-ci, pour réduire le temps de délai de l'action antidépressive des agents antidépresseurs actifs pharmaceutiquement acceptables de préférence choisis parmi le groupe constitué des agents ISRS, des IRSN (inhibiteurs de la recapture de la sérotonine et de la noradrénaline), des antagonistes du CRF, des antagonistes de NK1, des antagonistes de NK2, des antagonistes de NK3 et des combinaisons de ceux-ci.

7. Formulation ou système selon les revendications 1 ou 2, qui comprend des particules, des noyaux, des microparticules, des particules sphériques comprenant au moins des agents antidépresseurs actifs pharmaceutiquement acceptables de préférence choisis parmi le groupe constitué des agents ISRS, des IRSN (inhibiteurs de la recapture de la sérotonine et de la noradrénaline), des antagonistes du CRF, des antagonistes de NK1, des antagonistes de NK2, des antagonistes de NK3 et des combinaisons de ceux-ci et éventuellement au moins un composé choisi parmi le groupe constitué de l'acide acétylsalicylique, des sels et des esters de l'acide acétylsalicylique, de la diaspirine, et des mélanges de ceux-ci, lesdits particules, noyaux, microparticules, particules sphériques étant avantageusement pourvus d'un revêtement comprenant au moins un composé choisi parmi le groupe constitué de l'acide acétylsalicylique, des sels et des esters de l'acide acétylsalicylique, de la diaspirine, et des mélanges de ceux-ci, pour réduire le délai d'action des agents antidépresseurs actifs pharmaceutiquement acceptables de préférence choisis parmi le groupe constitué des agents ISRS, des IRSN (inhibiteurs de la recapture de la sérotonine et de la noradrénaline), des antagonistes du CRF, des antagonistes de NK1, des antagonistes de NK2, des antagonistes de NK3 et des combinaisons de ceux-ci, ledit revêtement étant essentiellement exempt des agents antidépresseurs actifs sélectifs pharmaceutiquement acceptables choisis de préférence parmi le groupe constitué des ISRS, des IRSN (inhibiteurs de la recapture de la sérotonine et de la noradrénaline), des antagonistes du CRF, des antagonistes de NK1, des antagonistes de NK2, des antagonistes de NK3 et des combinaisons de ceux-ci.

8. Formulation ou système selon les revendications 1 ou 2, qui est une forme solide comprenant de 4 mg jusqu'à 100 mg, de préférence de 4 à 20 mg des agents antidépresseurs actifs pharmaceutiquement acceptables choisis de préférence parmi le groupe constitué des ISRS, des IRSN (inhibiteurs de la recapture de la sérotonine et de la noradrénaline), des antagonistes du CRF, des antagonistes de NK1, des antagonistes de NK2, des antagonistes de NK3 et des combinaisons de ceux-ci.

9. Formulation ou système selon les revendications 1 ou 2, qui comprend de 5 à 40 mg d'acide acétylsalicylique, de sels ou d'esters de l'acide acétylsalicylique, de la diaspirine et des mélanges de ceux-ci, de préférence de 5 à 35 mg.

10. Formulation ou système selon les revendications 1 ou 2, qui comprend comme agent antidépresseur pharmaceutiquement acceptable une quantité efficace d'un agent choisi parmi le groupe constitué des ISRS, des IRSN (inhibiteurs de la recapture de la sérotonine et de la noradrénaline), des antagonistes du CRF, des antagonistes de NK1, des antagonistes de NK2, des antagonistes de NK3 et des combinaisons de ceux-ci.

11. Formulation ou système selon les revendications 1 ou 2, qui comprend comme agent antidépresseur pharmaceutiquement acceptable une quantité efficace d'un ou plusieurs agents ISRS.

12. Utilisation d'un composé choisi parmi le groupe constitué de l'acide acétylsalicylique, des sels et des esters de l'acide acétylsalicylique, de la diaspirine, et des mélanges de ceux-ci, pour la préparation d'une composition ou système antidépresseur avec un temps réduit de délai d'action antidépressive d'un agent antidépresseur actif choisi parmi le groupe constitué des ISRS, des IRSN (inhibiteurs de la recapture de la sérotonine et de la noradrénaline), des antagonistes du CRF, des antagonistes de NK1, des antagonistes de NK2, des antagonistes de NK3 et des combinaisons de ceux-ci.

13. Utilisation selon la revendication 12 pour la préparation d'une composition ou d'un système antidépresseur avec un facteur de risque de suicide inférieur associé à un agent antidépresseur actif choisi parmi le groupe constitué des ISRS, des IRSN (inhibiteurs de la recapture de la sérotonine et de la noradrénaline), des antagonistes du CRF, des antagonistes de NK1, des antagonistes de NK2, des antagonistes de NK3 et des combinaisons de ceux-ci.

14. Utilisation selon la revendication 12 pour la préparation d'une composition ou d'un système antidépresseur avec un temps d'action accru ou une plus longue durée d'efficacité d'un agent antidépresseur actif choisi parmi le groupe constitué des ISRS, des IRSN (inhibiteurs de la recapture de la sérotonine et de la noradrénaline), des antagonistes du CRF, des antagonistes de NK1, des antagonistes de NK2, des antagonistes de NK3 et des combinaisons de ceux-ci.
